# EUROPÄISCHE PATENTANMELDUNG

(11) **EP 1 562 046 A1**
(43) Veröffentlichungstag der Anmeldung: **10.08.2005**
(21) Anmeldenummer: 04002355.8
(22) Anmeldetag: 03.02.2004
(51) Int. Cl.: G01N 33/68, G01N 33/569, C12Q 1/28

(54) **Diagnose von Sepsis durch selektive Bestimmung der Konzentration der Cu/Zn Superoxiddismutase (Cu/Zn SOD) in Patientenproben**

(71) Anmelder: B.R.A.H.M.S Aktiengesellschaft, 16761 Hennigsdorf (DE)
(72) Erfinder: Bergmann, Andreas, Dr., 12351 Berlin (DE); Struck, Joachim, Dr., 12161 Berlin (DE); Ühlein, Monika, Dr., 10407 Berlin (DE); Morgenthaler, Nils G., 13503 Berlin (DE)
(74) Vertreter: Andrae, Steffen, Dr.

(57) **Zusammenfassung**

Verfahren zur frühzeitigen Ermittlung des Mortalitätsrisikos von Patienten auf Intensiv- oder Notfallstationen, bei dem man in einer Serum- oder Plasmaprobe eines solchen Patienten selektiv die Konzentration der Cu/Zn Superoxiddismutase (Cu/Zn SOD oder SOD-1) bestimmt und quantitativ oder semiquantitativ gemessene Konzentrationen, die über einem vorgegebenen Schwellenwert, z.B. über etwa 310 ng/ml, liegen, mit einem hohen Mortalitätsrisiko korreliert.

## Beschreibung

Die vorliegende Erfindung betrifft ein neues prognostisches Verfahren, das im Rahmen der klinischen Versorgung von Patienten auf Intensiv- und Notfallstationen, insbesondere Sepsispatienten, zur Anwendung kommen kann und Aussagen zum Mortalitätsrisiko bzw. zur Überlebenswahrscheinlichkeit solcher Patienten ermöglicht.

Die vorliegende Erfindung hat ihren Ausgangspunkt in intensiven Forschungsarbeiten der Anmelderin im Zusammenhang mit weiteren Verbesserungen der Diagnose und Therapie von Sepsis.

Als Entzündungen (Inflammationen) werden ganz allgemein bestimmte physiologische Reaktionen eines Organismus auf verschiedenartige äußere Einwirkungen wie z.B. Verletzungen, Verbrennungen, Allergene, Infektionen durch Mikroorganismen wie Bakterien und Pilze und Viren, auf Fremdgewebe, die Abstoßungsreaktionen auslösen, oder auf bestimmte entzündungsauslösende endogene Zustände des Körpers, z.B. bei Autoimmunerkrankungen und Krebs, bezeichnet. Entzündungen können als harmlose, lokal begrenzte Reaktionen des Körpers auftreten, sind jedoch auch typische Merkmale zahlreicher ernster chronischer und akuter Erkrankungen von einzelnen Geweben, Organen, Organ- und Gewebsteilen.

Lokale Entzündungen sind dabei meist Teil der gesunden Immunreaktion des Körpers auf schädliche Einwirkungen, und damit Teil des lebenserhaltenden Abwehrmechanismus des Organismus. Wenn Entzündungen jedoch Teil einer fehlgeleiteten Reaktion des Körpers auf bestimmte endogene Vorgänge wie z.B. bei Autoimmunerkrankungen sind und/oder chronischer Natur sind, oder wenn sie systemische Ausmaße erreichen, wie beim systemischen Inflammationssyndrom (Systemic Inflammatory Response Syndrome, SIRS) oder bei einer auf infektiöse Ursachen zurückzuführenden schweren Sepsis, geraten die für Entzündungsreaktionen typischen physiologischen Vorgänge außer Kontrolle und werden zum eigentlichen, häufig lebensbedrohlichen Krankheitsgeschehen von Patienten, die dementsprechend typischerweise auf der Intensivstation einer Klinik betreut werden müssen. In der folgenden Beschreibung werden derartige Patienten in der Regel als Sepsispatienten bezeichnet, wobei dieser Begriff jedoch nicht unbedingt eine vorausgehende Sepsisdiagnose bei den Patienten voraussetzt und generell kritisch kranke Patienten auf Intensivstationen, für die ein Sepsisrisiko besteht, erfassen soll.

Es ist heute bekannt, dass die Entstehung und der Verlauf von entzündlichen Prozessen von einer beträchtlichen Anzahl von Substanzen, die überwiegend proteinischer bzw. peptidischer Natur sind, gesteuert werden bzw. von einem mehr oder weniger zeitlich begrenzten Auftreten bestimmter Biomoleküle begleitet sind. Zu den an Entzündungsreaktionen beteiligten endogenen Substanzen gehören insbesondere solche, die zu den Cytokinen, Mediatoren, vasoaktiven Substanzen, Akutphasenproteinen und/oder hormonellen Regulatoren gezählt werden können. Die Entzündungsreaktion stellt eine komplexe physiologische Reaktion dar, an der sowohl das Entzündungsgeschehen aktivierende endogene Substanzen (z.B. TNF-α) als auch desaktivierende Substanzen (z.B. Interleukin-10) beteiligt sind.

Bei systemischen Entzündungen wie im Falle einer Sepsis bzw. des septischen Schocks weiten sich die entzündungsspezifischen Reaktionskaskaden unkontrolliert auf den gesamten Körper aus und werden dabei, im Sinne einer überschießenden Immunantwort, lebensbedrohlich. Zu den gegenwärtigen Kenntnissen über das Auftreten und die möglichen Rolle einzelner Gruppen endogener entzündungsspezifischer Substanzen wird beispielsweise verwiesen auf A.Beishuizen, et al., "Endogenous Mediators in Sepsis and Septic Shock", Advances in Clinical Chemistry, Vol.33, 1999, 55-131; und C.Gabay, et al., "Acute Phase Proteins and Other Systemic Responses to Inflammation", The New England Journal of Medicine, Vol.340, No.6, 1999, 448-454. Da sich das Verständnis von Sepsis, und damit auch die anerkannten Definitionen, in den letzten Jahren gewandelt haben, wird außerdem verwiesen auf K.Reinhart, et al., "Sepsis und septischer Schock", in: Intensivmedizin, Georg Thieme Verlag, Stuttgart.New York, 2001, 756-760; wo eine moderne Definition des Sepsis-Begriffes gegeben wird. Zur Bedeutung des Krankheitsbilds "schwere Sepsis" wird ferner verwiesen auf (34; Zahlen in Klammern beziehen sich in dieser Beschreibung auf die Literaturstellen, die unter der gleichen Nummer in der Literaturliste am Ende der Beschreibung erscheinen). Eine jüngere Zusammenfassung der Kriterien und Definitionen für eine Sepsis und eng verwandte Krankheitsbilder findet sich unter http://www.talessin.de/scripte/medizin/sepsisl.html. Im Rahmen der vorliegenden Anmeldung wird der Begriff Sepsis in einem umfassenden Sinne in Anlehnung an die Definitionen, wie sie den genannten Veröffentlichungen entnommen werden können, für septische Krankheitsbilder von schwer erkrankten Patienten auf Intensivstationen verwendet.

Während wenigstens im europäischen Raum die durch eine positive Blutkultur nachweisbare systemische bakterielle Infektion lange den Sepsisbegriff prägte, wird die Sepsis heute in erster Linie als systemische Entzündung verstanden, die infektiöse Ursachen hat, als Krankheitsgeschehen jedoch große Ähnlichkeiten mit systemischen Entzündungen aufweist, die durch andere Ursachen ausgelöst werden. Dem genannten Wandel des Sepsis-Verständnisses entsprechen Veränderungen der diagnostischen Ansätze. So wurde der direkte Nachweis bakterieller Erreger durch komplexe Überwachungen von Laborparametern und hämodynamischen Parametern unter Anwendung computergestützter sog. Score-Systeme (z.B. APACHE II SCORE; APACHE steht für "Acute Physiology and Chronic Health Evaluation"; vgl. (33) sowie Einleitung der DE 42 27 454 C1) sowie in jüngerer Zeit insbesondere auch durch den Nachweis bestimmter am Sepsisgeschehen bzw. am Entzündungsgeschehen beteiligter endogener Substanzen, d.h. spezifischer "Biomarker", ersetzt bzw. ergänzt.

Von der großen Zahl von Mediatoren und Akutphasenproteinen eignen sich dabei für diagnostische Zwecke insbesondere solche, deren Auftreten sehr spezifisch für Sepsis bzw. bestimmte Phasen einer Sepsis ist, deren Konzentrationen sich drastisch und diagnostisch signifikant verändern und die außerdem die für Routinebestimmungen erforderlichen Stabilitäten aufweisen und hohe Konzentrationswerte erreichen. Für diagnostische Zwecke steht dabei die zuverlässige Korrelation von Krankheitsgeschehen (Sepsis) mit dem jeweiligen Biomarker im Vordergrund, ohne dass dessen Rolle in der komplexen Kaskade der am Sepsisgeschehen beteiligten endogenen Substanzen stets im einzelnen bekannt sein muss.

Eine derartige als Sepsis-Biomarker besonders geeignete endogene Substanz ist Procalcitonin. Procalcitonin ist ein Prohormon, dessen Serum-Konzentrationen unter den Bedingungen einer systemischen Entzündung infektiöser Ätiologie (Sepsis) sehr hohe Werte erreichen, während es bei Gesunden so gut wie nicht nachweisbar ist. Hohe Werte an Procalcitonin werden außerdem in einem relativ frühen Stadium einer Sepsis erreicht, so dass sich die Bestimmung von Procalcitonin auch zu Früherkennung einer Sepsis und zur frühen Unterscheidung einer infektiös bedingten Sepsis von schweren Entzündungen, die auf anderen Ursachen beruhen, eignet. Die Bestimmung von Procalcitonin als Sepsismarker ist Gegenstand der Veröffentlichung M.Assicot, et al., "High serum procalcitonin concentrations in patients with sepsis and infection", The Lancet, vol.341, No.8844, 1993, 515-518; und der Patente DE 42 27 454 C2 bzw. EP 0 656 121 B1 bzw. US 5,639,617. Auf die genannten Patente und in der genannten Veröffentlichung angeführten frühen Literaturstellen wird zur Ergänzung der vorliegenden Beschreibung ausdrücklich Bezug genommen.

Die Verfügbarkeit des Sepsismarkers Procalcitonin hat der Sepsisforschung starke Impulse gegeben, und es werden gegenwärtig intensive Anstrengungen unternommen, weitere Biomarker zu finden, die die Procalcitonin-Bestimmung ergänzen können und/oder zusätzliche Informationen für Zwecke der Feindiagnostik bzw. Differentialdiagnostik zu liefern vermögen. Erschwert wird die Suche nach potentiellen neuen Sepsis-Biomarkern allerdings dadurch, dass häufig noch sehr wenig oder nichts über die genaue Funktion bzw. über die genauen Gründe für das Auftreten bestimmter endogener Substanzen, die am Sepsisgeschehen beteiligt sind, bekannt ist.

Die Ergebnisse der experimentellen Überprüfung eines fruchtbaren rein hypothetischen Ansatzes zur Ermittlung weiterer potentieller Sepsismarker finden sich in DE 198 47 690 A1 bzw. WO 00/22439. Dort wird gezeigt, dass bei Sepsis nicht nur die Konzentration des Prohormons Procalcitonin erhöht ist, sondern auch für andere Substanzen, die zu den Peptid-Prohormonen gerechnet werden können oder die Fragmente solcher Prohormone darstellen und eine für solche Prohormone typische Immunreaktivität aufweisen, signifikant erhöhte Konzentrationen beobachtet werden können. Während das beschriebene Phänomen gut dokumentiert ist, sind die genauen Ursachen für den nachweisbaren Anstieg der Konzentrationen von Prohormonen oder ihrer Fragmente bei Sepsis in den meisten Fällen weitgehend ungeklärt.

Die vorliegende Anmeldung baut auf ein Ergebnis eines anderen fruchtbaren, rein experimentellen Ansatzes für die Suche nach weiteren sepsisspezifischen Biomolekülen auf. Dieser beruht darauf, dass man durch Verabreichung eines Endotoxins an Primaten (Paviane) bei diesen eine künstliche Sepsis erzeugt und dann durch Vergleich der Gelelektrophorese-Proteinspotmuster von endotoxinbehandelten und von unbehandelten Pavianen endogene Substanzen von peptischer bzw. proteinischer Natur ermittelt, die nur bei den "septischen" Pavianen gefunden werden und die daher potentielle sepsisspezifische Biomarker darstellen. Das Primatenmodell wurde dabei aufgrund der sehr großen Ähnlichkeit der Physiologie von Primaten und Menschen und der hohen Kreuzreaktivität mit vielen therapeutischen und diagnostischen humanen Reagenzien gewählt.

Wie im experimentellen Teil älterer Patentanmeldungen der Anmelderin genauer ausgeführt wird, wird dabei nach experimenteller Auslösung einer künstlichen Sepsis in Pavianen durch Endotoxinverabreichung (LPS aus Salmonella Typhimurium) und 2D-gelektrophoretischer Aufarbeitung von Lebergewebe der behandelten Tiere eine Anzahl von nur bei den behandelten Tieren identifizierbaren Proteinspots gefunden. Die den Spots entsprechenden proteinischen Produkte werden aus dem Elektrophoresegel isoliert und massenspektrometrisch (v.a. mittels Tandem-Massenspektrometrie) untersucht.

Nach dem genannten Verfahren wurden, wie in älteren deutschen und europäischen Patentanmeldungen der Anmelderin erstmals beschrieben wurde, als neuartige Sepsismarker u.a. die Proteine "Inflammin" (WO 02/085937), CHP (WO 03/005035), lösliche Cytokeratin-1-Fragmente (sCY1F; WO 03/002600), das Proteine LASP-1 (WO 03/089934) sowie Enzyme wie Aldose-1-Epimerase (Mutarotase; WO 03/048780), Glycin-N-Acyl-Transferase (GNAT; WO 03/048781) und lösliche Carbamoylphosphat Synthetase 1 (CPS 1; WO 03/089933) identifiziert.

Der Inhalt der genannten älteren Anmeldungen der Anmelderin ist durch die ausdrückliche Bezugnahme auf diese Anmeldungen als Teil der Offenbarung der vorliegenden Anmeldung anzusehen.

Bei den geschilderten Untersuchungen wurden als sepsisspezifische Proteinspots auch zahlreiche Substanzen gefunden, die bereits als sog. "Akutphasenproteine" bekannt waren bzw. für die eine nachträgliche Literaturrecherche ergab, dass ihr Auftreten in einem Zusammenhang mit Entzündungen bzw. Sepsis bereits diskutiert wurde.

Ein solcher Proteinspot war auch der Spot, der sich bei seiner analytischen Aufarbeitung als ein Superoxiddismutase-Enzym, nämlich das Enzym Cu- und Zn-abhängige Superoxiddismutase (Cu/Zn SOD; SOD-1), erwies.

Superoxiddismutasen (SOD, EC 1.15.1.1.) sind Enzyme mit Antioxidantien-Funktion, die in der Lage sind, das reaktive Superoxidanion O₂⁻ in weniger reaktive Spezies umwandeln. Eukaryotische Zellen enthalten zwei unterschiedliche SOD-Typen, nämlich Cu/Zn SOD (auch als SOD-1 bekannt) und Mn SOD. Humane Cu/Zn SOD wird in erster Linie im Cytosol gefunden. Cu/Zn ist ein aus zwei identischen Untereinheiten bestehendes Dimeres mit einer molaren Masse von etwa 33 kDa. Humane Mn SOD (SOD-2), die vor allem in den Mitochondrien gefunden wird, ist ein Homotetramer und weist eine molare Masse von etwa 80 kDa auf. Außerdem wurde noch eine sog. extrazelluläre SOD oder EC-SOD identifiziert, die u.a. in extrazellulären Fluiden wie Plasma, Lymphe und Synovialfluid vorkommt. Die cDNA- bzw. Aminosäure-Sequenzen aller drei o.g. SOD Typen sind bekannt (vgl. z.B. (27), (28), (29)) und unterscheiden sich erheblich. Sie können einschlägigen Datenbanken (z.B. http://www.expasy.org/cgi-bin/niceprot) entnommen werden (Cu/Zn SOD oder SOD1: Swiss-Prot Accession Number: P00441; Mn SOD oder SOD2: Swiss-Prot Accession Number: P04179; extrazelluläre SOD oder EC-SOD bzw. SOD3; Swiss-Prot Accession Number: P08294). Wenn in der nachfolgenden Beschreibung nur die Abkürzung SOD verwendet wird, wird nicht zwischen den einzelnen SOD-Typen unterschieden, d.h. es geht in der Regel um die Diskussion von Befunden und Aussagen, bei denen die enzymatische Wirkung im Vordergrund steht.

Alle drei SOD Typen weisen aufgrund der unterschiedlichen Sequenzen ihrer Untereinheiten unterschiedliche Immunreaktivitäten auf und können mit Hilfe von nicht kreuzreagierenden Immunoassays selektiv bestimmt werden (vgl. z.B. (2)). Sowohl humane Cu/Zn SOD als auch humane Mn SOD wurden für therapeutische Zwecke/Versuche bereits in rekombinanter Form hergestellt (vgl. (5) und die darin zitierten weiterführenden Literaturstellen).

Insbesondere zu SOD-1 und SOD-2 existiert eine umfangreiche wissenschaftliche Literatur, die jedoch ein komplexes und teilweise widersprüchliches Bild des physiologischen Vorkommens und der Rolle der verschiedenen SOD Typen bei verschiedenen Krankheitszuständen, in verschiedenen Spezies und Organen bzw. Geweben und unter verschiedenen äußeren Einflüssen liefert. U.a. ist seit langem bekannt, dass die Expression bzw. Translation von SOD durch Endotoxine (LPS) beeinflußbar ist, und dass die LPS-Wirkung zumindest bei Ratten u.a. vom Sauerstoffanteil in der Atemluft abhängt (vgl. z.B. (1) und (3)). Die experimentellen Befunde unterscheiden sich jedoch beträchtlich, je nach Herkunft des untersuchten Gewebes bzw. der untersuchten Zellkultur sowie in Abhängigkeit davon, ob man die mRNA- oder die Protein-Ebene betrachtet, wobei außerdem das Zeitintervall der Beobachtung eine zusätzliche Rolle spielt (vgl. z.B. (1) bis (4); (6), (7), (9), (10), (12), (13), (14)). Es kann jedoch verallgemeinernd gesagt werden, dass in den meisten der Fälle von einer Steigerung der Mn SOD Expression/Translation unter dem Einfluss von LPS berichtet wird, während eine entsprechende Wirkung im Hinblick auf Cu/Zn SOD nicht oder nur in einer deutlich schwächeren Ausprägung beobachtbar war. In den meisten Arbeiten wird sogar von einer Cu/Zn SOD Abnahme under der Einfluss von LPS berichtet (vgl. z.B. (1) bis (4), (7), (9), (10), (12) bis (14)). Die erhöhte Bildung von SOD bei verschiedenen entzündlichen Erkrankungen wurde als Teil eines Schutzmechanismus gegen reaktive Sauerstoffspezies (ROS) diskutiert, und SOD wurde demgemäß auch als Therapeutikum u.a. zur Entzündungshemmung (vgl. (5)) eingesetzt.

Die Tatsache, dass viele der Ergebnisse mit verschiedenen Tiermodellen erhalten wurden, verkompliziert das Bild zusätzlich. So waren bei Ratten beobachtete schützende Effekte einer LPS-Verabreichung z.B. bei Rindern nicht verifizierbar (vgl. z.B. (13) und die darin angeführten weiterführenden Literaturstellen). Es herrscht aber Einigkeit darüber, dass SOD u.a. im Zusammenhang mit Sauerstoffstress und Entzündungsreaktionen des Körpers eine wichtige physiologische Rolle spielt.

Demzufolge wurde SOD auch schon im Zusammenhang mit Sepsis bzw. sepsistypischen Komplikationen diskutiert. In der Veröffentlichung (8) bzw. einer daran anknüpfenden Patentanmeldung WO 94/22016 A1 wird berichtet, dass Sepsispatienten erhöhte Spiegel an Mn SOD aufweisen, und dass bei Mn SOD Spiegeln oberhalb eines bestimmten Schwellenwerts das Risiko einer Entwicklung der als Komplikation gefürchteten akuten respiratorischen Insuffizienz (ARDS; adult respiratory distress syndrome) stark erhöht ist. Mn SOD wurde dabei immunologisch mittels eines selektiven ELISA Assays gemessen.

Auf diesen Ergebnissen aufbauend und auf diese Bezug nehmend wurde die Rolle von SOD bei Sepsis in der Folge mehrfach untersucht, und zwar u.a. auch in der Hoffnung, durch die SOD-Bestimmung frühzeitig Anhaltspunkte für den zu erwartenden Verlauf einer Sepsis zu erhalten (vgl. (11), (15) bis (17), (19), (20)). In allen einschlägigen Untersuchungen erfolgte jedoch keine selektive Bestimmung einzelner SOD-Typen, sondern es wurde pauschal die SOD-Enzymaktivität mit Verfahren ((30), (31)) bestimmt, die keine Aussage darüber ermöglichen, auf welchen SOD-Typ die in der Probe festgestellte Enzymaktivität zurück zu führen ist. Vor dem Hintergrund der o.g. Basisveröffentlichung gemäß (8) bzw. WO 94/22016 A1 mußte jedoch davon ausgegangen werden, dass die gemessene SOD-Enzymaktivität mindestens überwiegend auf erhöhte Mn SOD- und möglicherweise zusätzlich EC-SOD-Werte zurück zu führen ist. Eine spezifische Rolle der Cu/Zn SOD, oder eine besondere Nützlichkeit der selektiven Bestimmung von Cu/Zn SOD, wurde im Zusammenhang mit Sepsis in keiner der o.g. Veröffentlichungen diskutiert. Es ist in diesem Zusammenhang anzumerken, dass die Messung einer Enzymaktivität nicht mit der Messung der Konzentration eines für die enzymatische Wirkung verantwortlichen Biomoleküls, oder einer Gruppe solcher Biomoleküle, gleichgesetzt werden kann, da in den untersuchten Proben die Enzymwirkung z.B. gehemmt oder verstärkt sein kann, ohne dass sich die Konzentration des Enzyms/der Enzyme notwendigerweise ändern muss. Für SOD wurde z.B. eine Verminderung des Enzymwirkung durch reaktive Stickstoffspezies (RNS) beschrieben (24).

Auch in jüngeren Veröffentlichungen, die zeigen, dass die Expression des Cu/Zn SOD-Gens durch Stickoxid gesteuert zu sein scheint ((14), (18)), wird keine Beziehung zwischen der in einer Probe eines septischen Patienten messbaren Konzentration der Cu/Zn SOD und dem zu erwartenden Verlauf einer Sepsis hergestellt. Die erhöhte Cu/Zn SOD Expression wird als Schutzmechanismus diskutiert.

Angesichts des klaren Nachweises von Cu/Zn SOD im Pavian-Sepsismodell der Anmelderin und angesichts der Tatsache, dass die in der Literatur zu findenden Angaben zum Auftreten von SOD bei Sepsis im Hinblick auf die Rolle von humaner SOD im Rahmen des Sepsisgeschehens und die diagnostische Nützlichkeit einer spezifischen Bestimmung von humaner Cu/Zn SOD in Serum- bzw. Plasmaproben von Sepsispatienten keinerlei sichere Anhaltspunkte lieferten, entschloss sich die Anmelderin, durch Vermessung von Serum- bzw. Plasmaproben eines großen Kollektivs von Sepsispatienten zu überprüfen, ob irgendeine Beziehung zwischen den messbaren Konzentrationen von humaner Cu/Zn SOD in den Proben und dem dokumentierten Krankheitsverlauf der Patienten, von denen die untersuchten Proben stammten, besteht.

Dahinter stand der schmerzlich empfundene Mangel, dass trotz des inzwischen in der klinischen Praxis eingeführten und bewährten diagnostischen Sepsismarkers Procalcitonin weiterhin ein dringender Bedarf nach ergänzenden Biomarkern für die Sepsis-Feindiagnostik und insbesondere für eine frühzeitige Sepsisprognostik besteht, die ein diagnostisches und vor allem prognostisches Potential aufweisen, das die klinische kontinuierliche Multiparameter-Überwachung von Patienten auf Intensivstationen (z.B. gemäß APACHE II SCORE) sinnvoll ergänzen kann oder einer solchen Überwachung ggf. sogar gleichwertig ist.

Überraschender Weise ergaben die von der Anmelderin durchgeführten Messungen von Cu/Zn SOD in Serum- bzw. Plasmaproben von Patienten von Intensiv- und Notfallstationen, dass die gemessenen Werte eine hervorragende Korrelation mit dem beobachteten Krankheits- bzw. Sepsisverlauf aufweisen und insbesondere eine sichere frühzeitige Zuordnung kritisch erkrankter Patienten zu den Gruppen der wahrscheinlich überlebenden bzw. nicht überlebenden Patienten erlaubt. Die quantitative Bestimmung von Cu/Zn SOD zeigte mit bemerkenswerter, von keinem anderen untersuchten biochemischen Marker übertroffener diagnostischer Sensitivität und Spezifität die Überlebenswahrscheinlichkeit von Patienten an, die - in der Regel mit Sepsisverdacht oder Sepsisdiagnose - auf die Intensivstation eingeliefert wurden. Sie erlaubt damit die Identifizierung einer besonderen Patienten-Risikogruppe unter Sepsispatienten.

Demgemäß betrifft die vorliegende Erfindung gemäß Anspruch 1 ein Verfahren zur frühzeitigen Ermittlung des Mortalitätsrisikos von Patienten auf Intensiv- ud Notfallstationen, bei dem man in einer Serum- oder Plasmaprobe eines solchen Patienten selektiv die Konzentration der Cu/Zn Superoxiddismutase (Cu/Zn SOD oder SOD-1) bestimmt und - quantitativ oder semiquantitativ - gemessene Konzentrationen, die über einem vorgegebenen Schwellenwert liegen, mit einem hohen Mortalitätsrisiko (einer niedrigen Überlebenswahrscheinlichkeit) korreliert.

Vorteilhafte oder fachgebietsübliche Ausgestaltungen eines solchen Verfahrens sind Gegenstand der Ansprüche 2 bis 12.

Nachfolgend wird das erfindungsgemäße Verfahren unter Bezugnahme auf Messergebnisse und diese Messergebnisse veranschaulichende graphische Darstellungen noch näher erläutert.

Die Figuren zeigen:
- Figur 1: die Werte der Messung der Cu/Zn SOD-Konzentrationen für ein Kollektiv von Sepsispatienten, wobei die Messungen mit Hilfe eines für Cu/Zn SOD spezifischen Enzym-Immunoassays erfolgten. Die Proben wurden unter Berücksichtigung der zugehörigen Dokumentation des Krankheitsverlaufs einer Gruppe der Exitus-Patienten und einer Gruppe der Überlebenden zugeordnet.
- Fig. 2: die Ergebnisse der Messungen der enzymatischen SOD-Aktivität für gleichen Proben wie in Fig.1, in der gleichen Aufteilung auf Exitus-Patienten und Überlebende;
- Fig. 3: eine Auswertung der in den Fig. 1 und 2 gezeigten Messergebnisse in der Form eines ROC-Plots, wobei die Ergebnisse der immunchemischen Messungen der Cu/Zn SOD (durchgezogene fette Linie) den Ergebnissen der Messung der SOD-Enzymaktivität (punktierte Linie) gegenüber gestellt sind.

Wie sich aus den nachfolgenden Beispielen ergibt, wurden die immunchemischen Messungen der Cu/Zn SOD in Plasmen von Patienten von Intensivstationen (Sepsispatienten) mit einem für Forschungszwecke kommerziell erhältlichen ELISA-Assay (31) nach den Anweisungen des Herstellers durchgeführt.

Es versteht sich jedoch, dass, die erforderliche Spezifität und Empfindlichkeit vorausgesetzt, auch irgendwelche anderen bekannten oder nach bekannten Prinzipien arbeitenden Immunoassays zur Bestimmung von Cu/Zn SOD eingesetzt werden können. Beispiele für derartige Assays finden sich u.a. in (2) und Patentanmeldungen wie EP 217 542 A2, EP 327 337 A2 und WO 90/06513 A1.

Grundsätzlich können alle für die selektive quantitive oder wenigstens semiquantitative Bestimmung von Cu/Zn SOD geeigneten immunchemischen Verfahren für die Messung eingesetzt werden.

Bei einer bevorzugten Ausführungsform wird das Verfahren als heterogener Sandwich-Immunoassay durchgeführt, bei dem ein für Cu/Zn SOD spezifischer Antikörper an eine beliebige Festphase, beispielsweise die Wände beschichteter Teströhrchen (z.B. aus Polystyrol; "Coated Tubes"; CT) oder an Mikrotiterplatten, zum Beispiel aus Polystyrol, oder an Partikel, beispielsweise Magnetpartikel immobilisiert ist, während ein weiterer Antikörper einen Rest trägt, der ein direkt nachweisbares Label darstellt oder eine selektive Verknüpfung mit einem Label ermöglicht und der Detektion der gebildeten Sandwich-Strukturen dient. Auch eine zeitlich verzögerte bzw. nachträgliche Immobilisierung unter Verwendung geeigneter Festphasen ist möglich.

Grundsätzlich können alle in Assays der beschriebenen Art verwendbaren Markierungstechniken angewandt werden, zu denen Markierungen mit Radioisotopen, Enzymen, Fluoreszenz-, Chemolumineszenz- oder Biolumineszenz-Labeln und direkt optisch detektierbaren Farbmarkierungen, wie beispielsweise Goldatomen und Farbstoffteilchen, wie sie insbesondere für sog. Point-of-Care (POC) oder Schnelltests verwendet werden, gehören. Es liegt somit im Rahmen der vorliegenden Erfindung, das erfindungsgemäße Verfahren auch als Schnelltest auszugestalten.

Die beiden Antikörper können auch im Falle heterogener Sandwich-Immunoassays Teile eines Nachweissystems der nachfolgend im Zusammenhang mit homogenen Assays beschriebenen Art aufweisen. Das erfindungsgemäße Verfahren kann somit beispielsweise auch unter Anwendung eines homogenen Nachweisverfahrens durchgeführt werden, bei dem die aus den beiden Antikörpern und der nachzuweisenden Cu/Zn SOD gebildeten Sandwichkomplexe in der flüssigen Phase suspendiert bleiben. In einem solchen Fall ist es bevorzugt, beide Antikörper mit Teilen eines Nachweissystems zu markieren, das dann, wenn beide Antikörper in einen einzigen Sandwich integriert werden, eine Signalerzeugung oder Signalauslösung ermöglicht. Derartige Techniken sind insbesondere als Fluoreszenzverstärkungs- oder Fluoreszenzlöschungs-Nachweisverfahren ausgestaltbar. Ein besonderes bevorzugtes derartiges Verfahren betrifft die Verwendung von paarweise einzusetzenden Nachweisreagenzien, wie sie beispielsweise beschrieben sind in US-A-4 822 733, EP-B1-180 492 oder EP-B1-539 477 und dem darin zitierten Stand der Technik. Sie ermöglichen eine Messung, die selektiv nur Reaktionsprodukte erfaßt, die beide Markierungskomponenten in einem einzigen Immunkomplex enthalten, direkt in der Reaktionsmischung. Als Beispiel ist auf die unter den Marken TRACE® (Time Resolved Amplified Cryptate Emission) bzw. KRYPTOR® angebotene Technologie zu verweisen, die die Lehren der o.g. Anmeldungen umsetzt.

In dem nachfolgenden Beispiel wurde als bevorzugter Schwellenwert (Cut-off) für die Prognosen "100% Mortalitätsrisiko" bzw. "hohe Überlebenswahrscheinlichkeit" ein Wert von 310 ng/ml ermittelt. Dieser Schwellenwert kann jedoch in Abhängigkeit vom prognostischen Ziel variiert werden. Es ist ferner anzumerken, dass der im Beispiel angegebene optimale Schwellenwerte mit dem kommerziell erhältlichen Assay gemäß (31) ermittelt wurde. Schwellenwerte sind jedoch stets abhängig von der Eichung des zur Bestimmung verwendeten immunchemischen Verfahrens. Wird ein anderer Immunoassay verwendet, können sich andere absolute Schwellenwerte für die messbaren Cu/Zn Konzentrationen ergeben. Eine Anpassung an den hierin angegebenen Schwellenwert durch eine geeignete Eichung des verwendeten speziellen Assays mit dem Assay gemäß (31) sollte jedoch stets möglich sein. Soweit ein o.g. Schwellenwert in den Patentansprüchen erscheint, ist er im o.g. Sinne zu verstehen und kann patentrechtlich nicht als absolutes Kriterium für eine Anwendung des erfindungsgemäßen Verfahrens interpretiert werden.

Die in einer konkreten klinischen Umgebung verwendeten Schwellenwerte sind somit eichungsabhängig. Dem medizinischen Praktiker bereit dieser Umstand jedoch keine Probleme.

### Versuchsergebnisse:

### 1. Materialien und Methoden:

Nachfolgend wird die statistische Auswertung der Messung der Cu/Zn SOD-Konzentrationen in Plasmen von Patienten von Intensivstationen beschrieben. Jede der vermessenen Proben war nach der Probennahme (Venenblut) sofort auf fachübliche Weise unter Gewinnung eines EDTA-Plasmas aufgearbeitet worden, und die EDTA-Plasma-Probe anschließend sofort bei - 20°C eingefroren worden. Die eingefrorenen Plasmen wurden wie die zugehörigen Dokumentationen des Krankheitsverlaufs für Messungszwecke aufbewahrt. Die aufgetauten Proben wurden außer für die nachfolgend beschriebenen Bestimmungen auch im Rahmen verschiedener anderer Bestimmungen verwendet, die im vorliegenden Zusammenhang nicht näher erläutert werden müssen.

Die Bestimmung der Cu/Zn SOD-Konzentrationen in den einzelnen Plasmen erfolgte unter Verwendung eines kommerziell für Forschungszwecke erhältlichen ELISA-Assays ("human Cu/Zn SOD ELISA BMS222", Bender MedSystems, MedSystems Diagnostics GmbH, Rennweg 95b, A-1030 Wien, Österreich) nach der Arbeitsvorschrift der Hersteller im zugehörigen Manual (32).

Die zu Vergleichszwecken durchgeführte Bestimmung der enzymatischen SOD-Aktivität in den Proben erfolgte analog (11) nach dem Verfahren gemäß (30).

Zur statistischen Auswertung der Messungen wurde die entsprechende handelsübliche Software (Prism 4.0, Graphpad.com) verwendet.

### 2. Messungen von Plasmen eines Patientenkollektivs

### 2a. Primärmessungen

Um für die nachfolgenden Messungen Bezugswerte zu erhalten, wurden die Cu/Zn SOD-Konzentrationen in Proben von gesunden Probanden gemessen. Die Werte streuten erheblich im Bereich von 114,1 ng/ml bis 352,1 ng/ml um einen Medianwert von 224 ng/ml (Standard-Abweichung 49,70 ng/ml). Es wurden keine Unterschiede zwischen männlichen und weiblichen Probanden beobachtet.

### 2b. Cu/Zn SOD und das Mortalitätsrisiko bzw. die Überlebenswahrscheinlichkeit von Intensivstationspatienten mit den Diagnosen Sepsis, schwere Sepsis und septischer Schock

Für die nachfolgenden Messungen wurden 103 Plasmaproben einer bei der Anmelderin verfügbaren Bank von Proben von Sepsispatienten (Diagnose Sepsis, schwere Sepsis und septischer Schock) von verschiedenen Intensivstationen verwendet. Die Blutabnahme erfolgte innerhalb der ersten 48 h nach Aufnahme auf die Intensivstation.

Die Messungen der Cu/Zn SOD-Konzentrationen und die Auswertung der Messungen erfolgte wie erläutert. Im Hinblick darauf, dass in (8) im Zusammenhang mit Sepsis bzw. der Entwicklung von ARDS die Bestimmung von Mn SOD vorgeschlagen wird, und darauf aufbauend in (11) die Bestimmung der SOD-Enzymaktivität in Proben von Sepsispatienten als Prognosemarker diskutiert wird, wurden die für Cu/Zn SOD erhaltenen Messwerte und deren prognostische Signifikanz gemäß der vorliegenden Erfindung verglichen mit den Ergebnissen, die für die gleichen Proben bei einer Bestimmung der SOD-Enzymaktivität (kU/L) nach dem in (11) angewandten Verfahren gemäß (30) erhalten werden.

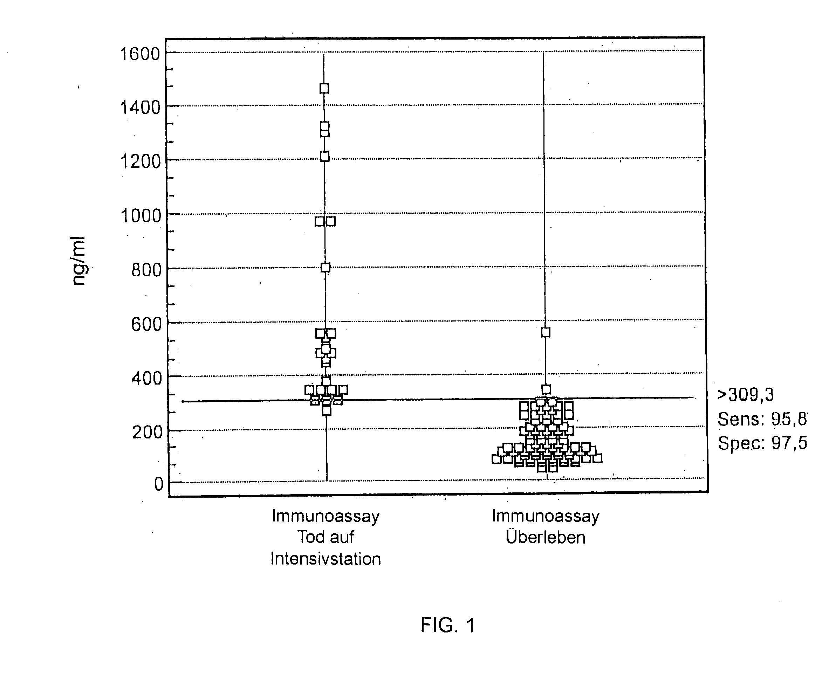

Die Ergebnisse der beiden Arten von Messungen sind in den Figuren 1, 2 und 3 gezeigt. Auf der Basis einer Gruppierung der untersuchten Proben in solche von Exitus-Patienten (79 Patienten) und von Überlebenden (24 Patienten) wurde als geeigneter Schwellenwert (Cut-Off) ein Wert von etwa 310 ng/ml ermittelt und für die Auswertung benutzt.

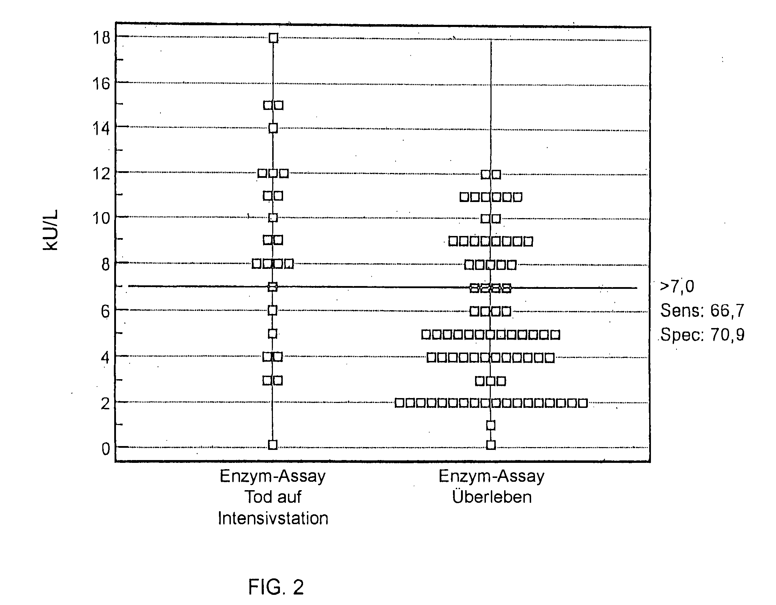

Ein Vergleich der Fig. 1 und 2 veranschaulicht, dass eine immunchemische Bestimmung der Cu/Zn SOD-Konzentrationen im Plasma von Patienten auf Intensivstationen (Sepsispatienten) nicht mit einer Bestimmung einer SOD-Enzymaktivität in den gleichen Proben gleichgesetzt werden kann, da qualitativ ganz andere Ergebnisse erhalten werden.

Außerdem wurden die Ergebnisse der Messungen der Cu/Zn SOD-Konzentrationen sowie der zu Vergleichszwecken durchgeführten Messungen der enzymatischen Aktivität mittels sogenannter ROC-Kurven (Receiver Operating Characteristic Plots) ausgewertet, die die Sensivitäten und Spezifitäten der Cu/Zn SOD-Bestimmung für das Patientenkollektiv in Relation setzen. Die Sensitivität wurde berechnet als Anteil richtig erkannter später Verstorbener bezogen auf alle später Verstorbenen. Die Spezifität wurde berechnet als Anteil der richtig erkannten Überlebenden bezogen auf die Gesamtzahl der Überlebenden.

Bei ROC-Kurven kann die Fläche zwischen der betreffenden Kurve und einer unter einem Winkel von 45° verlaufenden Geraden ("area under the ROC function" oder "area under the curve", AUC) als Kenngröße für die statistische Relevanz einer Bestimmung genommen werden. Je größer diese Fläche ist, desto höher ist die diagnostische bzw. prognostische Signifikanz.

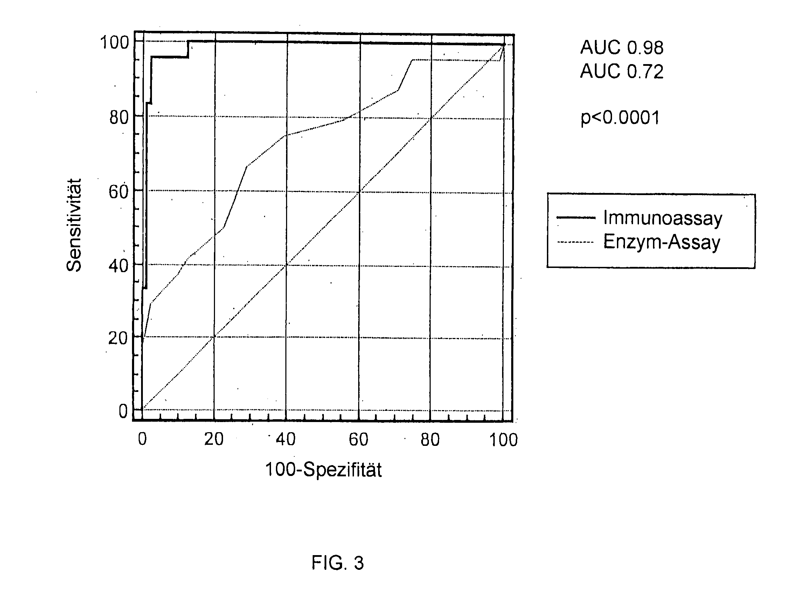

Bei einer ROC-Auswertung der Werte gemäß den Fig. 1 und 2 zeigt sich gemäß Fig. 3, dass die spezifische immunchemische Bestimmung der Cu/Zn SOD (fette durchgezogene Linie) sehr viel aussagekräftigere und signifikatere Ergebnisse liefert als eine Bestimmung der SOD-Enzymaktivität (punktierte Linie) in den gleichen Proben.

Aufgrund der o.g. Ergebnisse der statistischen Auswertung der Messung von Cu/Zn SOD in Plasmen von Patienten von Intensivstationen eröffnet sich überraschender Weise eine neue Möglichkeit, durch Messung der Konzentration dieses Biomarkers Cu/Zn SOD bei Patienten, die auf eine Intensivstation eingeliefert werden, eine rasche Voraussage zu deren Überlebenschancen zu erhalten. Eine solche Messung ist insbesondere in Kombination mit einer Messung von Procalcitonin zur Sepsisdiagnose sinnvoll. Patienten mit hohen Cu/Zn SOD-Konzentrationen stellen sich dann als besondere Risikogruppe unter den Sepsispatienten dar, die eine besondere Behandlung und therapeutische Intervention rechtfertigen. In diesem Zusammenhang ist z.B. darauf hinzuweisen, dass kürzlich in den U.S.A. eine Zulassung von aktiviertem Protein C (Drotrecogin oder Xigris von Eli Lilly) zur Therapie von Sepsis erfolgt ist, jedoch nur für diejenige Teilpopulation der Sepsispatienten, für die ein hohes Mortalitätsrisiko besteht. Es stellt sich somit als Herausforderung die Frage, wie diese Teilpopulation korrekt ermittelt werden kann. Die mangels Alternativen vorgesehene Anwendung des APACHE II SCORES wird in der Literatur kontrovers diskutiert (35), (36), (37). Die Verfügbarkeit des Prognosemarkers Cu/Zn SOD als zusätzliches oder alternatives Instrument zur Identifizierung von Sepsis-Hochrisikopatienten stellt in diesem Zusammenhang einen wichtigen Fortschritt dar.

### Literaturliste:

1. Michael A. Hass et al., The Effect of Bacterial Endotoxin on Synthesis of (Cu,Zn)Superoxide Dismutase in Lungs of Oxygen-exposed Rats, in: J.Biol.Chem. 257, 9379-9383, 1982
2. K. Asayama et al., Selective induction of manganous superoxide dismutase in human monocytes, in: Am.J.Physiol. 249, C393-C397, 1985
3. Jawaid Iqbal et al., Endotoxin increases lung Cu,Zn superoxide dismutase mRNA: O₂ raises enzyme synthesis, in: Am.J.Physiol 257, L61-L64, 1989
4. Gary A. Visner et al., Regulation of Manganese Superoxide Dismutase by Lipopolysaccharide, Interleukin-1, and Tumor Necrosis Factor, in: J.Biol.Chem. 265, 2856-2864, 1990
5. Marian Gorecki et al., Recombinant Human Superoxide Dismutases: Production and Potential Therapeutical Uses, in: Free Rad. Res. Comms., Vols. 12-13, 401-410, 1991
6. Xue-Jun Kong et al., Regulation of Cu,Zn-Superoxide Dismutase in Bovine Pulmonary Artery Endothelial Cells, in: J.Cell.Physiol., 153:491-497 (1992)
7. Lester S. Gibbs et al., Mn and Cu/Zn SOD Expression in Cells from LPS-sensitive and LPS-resistant Mice, in: Free Rad. Biol. Med. Vol.12, 107-11, 1992
8. Jonathan A. Leff et al., Serum Antioxidants as predictors of adult respiratory distress syndrome in patients wit sepsis, in: Lancet 1993; 341:777-780
9. Kenji Mokuno et al., Induction of Manganese Superoxide Dismutase by Cytokines and Lipopolysaccharides in Cultured Mouse Astrocytes, in: J.Neurochem. 63, 612-616 (1994)
10. Reishi ABE et al., Lipopolysacharide Induces Manganese Superoxide Dismutase in the Rat Pancreas: Its Role in Caerulin Pancreatitis, in: Biochem.Biophys.Res.Comm., Vol. 217, No.3, 1216-1222, 1995
11. Ann Warner et al., Prognostic Role of Antioxidant Enzymes in Sepsis: Preliminary Assessment, in: Clin.Chem. 41/6, 867-871 (1995)
12. Bidyut Ghosh et al., Tissue Differences in Antioxidant Enzyme Gene Expression in Response to Endotoxin, in: Free Rad. Biol. Med., Vol.21, No.4, 533-540, 1996
13. Mary Leach et al., Decline in the expression of copper/zinc superoxide dismutase in the kidney of rats with endotoxin shock: Effects of the superoxide anion radical scavenger, tempol, on organ injury, in: Br. J. Pharmacol., 125, 817-825 (1998)
14. Stefan Frank et al., Identification of copper/zinc superoxide dismutase as a novel nitric oxide-regulated gene in rat glomerular mesangial cells and kidneys of endotoxemic rats, in: FASEB J. 13, 869-882 (1999)
15. Seema et al., Serum TNF-Alpha and Free Radical Scavengers in Neonatal Septicemia, in: Indian J Pediatr. 1999; 66:511-516
16. N.K. Dubey et al., Free Oxygen radicals in acute renal failure, in: Indian Pediatrics 2000; 37:153-158
17. Simmi Kharb et al., Role of Oxygen Free Radicals in Shock; JAPI 2000; 48:956-957
18. Stefan Frank et al., Identification of copper/zinc dismutase as a nitric oxide-regulated gene in human (HaCaT) keratinocytes: Implications for keratinocyte proliferation, in: Biochem.J. (2000) 346, 719-728
19. Sanjay Batra et al., Alterations in antioxidant status during neonatal sepsis, in: Ann.Trop.Paediatrics (2000) 20, 27-33
20. P.Bela et al., Oxidative stress status: possible guideline for clinical management of critically ill patients; in: Panminerva Med 2001, 43(1):27-31
21. Yasuda M. et al., Prognostic significance of serum superoxide dismutase activity in patients with gastric cancer, in: Gastric Cancer 2002; 5(3): 148-53
22. Taysi S, et al., Lipid peroxidation, some extracellular antioxidants, and antioxidant enzymes in serum of patients with rheumatoid arthritits, in: Rheumatol Int 2002 21(5): 200-204;
23. Taysi S et al., Serum oxidant/antioxidant status of patients with systemic lupus erythematosus, Clin Chem Lab Med 2002, 40(7):684-688
24. John M. Lawler et al., Specificity of antioxidant enzyme inhibition in skeletal muscle to reactive nitrogen species donors, in: Biochem.Biophys.Res.Commun. 294(2002) 1093-1100
25. Paolo Mondola et al., The Cu,Zn superoxide dismutase in neuroblastoma SK-N-BE cells is exported by a microvesicles dependent pathway, in: Mol.Brain Res. 110(2003) 45-51
26. Moshe Marikovsky et al., Cu/Zn Superoxide Dismutase Plays Important Role in Immune Response; J.Immunol., 2003, 170:2993-3001
27. Barra D., et al., The complete amino acid sequence of human Cu/Zn superoxide dismutase; FEBS Letters 120, 53-56 (1980)
28. Beck Y. et al., Human Mn superoxide dismutase cDNA sequence; Nucleic Acids Res. 16:6224 (1988)
29. Hjalmarsson K. et al., Isolation and sequence of complementary DNA encoding human extracellular superoxide dismutase; Proc. Natl. Acad, Sci. U.S.A. 84:6340-6344 (1987)
30. Joseph BZ et al., Activities of SODs and NADPH oxidase in neutrophils obtained from asthmatic and normal donors; Inflammation 1993; 174:331-336
31. Mishra HP et al., The role of superoxide anion in the antioxidation of the epinephrine and simple assay for superoxide dismutase; J.Biol.Chem. 1971; 247:3170-3175
32. Product Information and Manual: human Cu/Zn SOD ELISA BMS222, Bender MedSystems, MedSystems Diagnostics GmbH, Rennweg 95b, A-1030 Vienna, Austria (3.9.1997)
33. G.Pilz et al., Krankenpflege-Journal 29 (1991), S.483-492
34. Niels C. Riedemann et al., The enigma of sepsis, J.Clin.Invest. 112:460-467 (2003)
35. Manns Bj et al., An economic evalutation of activated protein C treatment for severe sepsis, N Engl J Med 2002; 347:993-1000
36. Warren HS et al., Risks and benefits of activated protein C treatment for severe sepsis, N Engl J Med 2002; 347:1027-1030
37. Siegel JP, Assessing the use of activated protein C in the treatment of severe spesis, N Engl J Med 2002; 347:1030-1034

## Patentansprüche

1. Verfahren zur frühzeitigen Ermittlung des Mortalitätsrisikos von Patienten auf Intensiv- oder Notfallstationen, **dadurch gekennzeichnet, dass** man in einer Serum- oder Plasmaprobe eines solchen Patienten selektiv die Konzentration der Cu/Zn Superoxiddismutase (Cu/Zn SOD oder SOD-1) bestimmt und Konzentrationen, die über einem vorgegebenen Schwellenwert liegen, mit einem hohen Mortalitätsrisiko korreliert.

2. Verfahren nach Anspruch 1, **dadurch gekennzeichnet, dass** die Patienten Intensivstations-Patienten sind, für die die klinische Diagnose auf Sepsis, schwere Sepsis oder septischer Schock lautet.

3. Verfahren nach Anspruch 1 oder 2, **dadurch gekennzeichnet, dass** das Verfahren zur Bestimmung der Cu/Zn SOD-Konzentrationen ein für Cu/Zn SOD selektives immunchemisches Bestimmungsverfahren ist.

4. Verfahren nach Anspruch 3, **dadurch gekennzeichnet, dass** das selektive immunchemische Bestimmungsverfahren eine Ligandenbindungsassay vom kompetitiven Typ oder Sandwichtyp ist.

5. Verfahren nach einem der Ansprüche 1 bis 4, **dadurch gekennzeichnet, dass** man die Korrelation zwischen der in der Serum- oder Plasmaprobe vorhandenen Cu/Zn SOD Konzentration und dem Schwellenwert durch eine quantitative oder semiquantitative Konzentrationsbestimmung herstellt.

6. Verfahren nach Anspruch 4 oder 5, **dadurch gekennzeichnet, dass** der Ligandenbindungsassay eine homogener oder heterogener Immunoassay vom Sandwichtyp ist, bei dem zum Nachweis von Cu/Zn SOD wenigstens ein markierter monoklonaler oder polyklonaler Antikörper verwendet wird und die Markierung ausgewählt ist aus Radioisotop-, Fluoreszenz-, Chemilumineszenz-, Enzym- und direkten optisch detektierbaren Farbstoffpartikeln.

7. Verfahren nach einem der Ansprüche 1 bis 6, **dadurch gekennzeichnet, dass** man als optimalen Schwellenwert für die gemessene Cu/Zn SOD-Konzentration, einen Wert von 310 ng/ml oder mehr wählt.

8. Verfahren nach einem der Ansprüche 1 bis 7, **dadurch gekennzeichnet, dass** es im Rahmen einer Multiparameter-Bestimmung durchgeführt wird, bei der gleichzeitig eine quantitatvie oder qualitative Bestimmung mindestens eines weiteren Sepsis-Prognoseparameters erfolgt.

9. Verfahren nach Anspruch 8, **dadurch gekennzeichnet, dass** im Rahmen der Multiparameter-Bestimmung neben Cu/Zn SOD wenigstens ein weiterer Parameter bestimmt wird, der ausgewählt ist aus der Gruppe, die besteht aus Procalcitonin, CA 19-9, CA 125, S100B, S100A-Proteinen, löslichen Cytokeratin-Fragmenten, insbesondere CYFRA 21, TPS und/oder löslichen Cytokeratin-1-Fragmenten (sCY1F), den Peptiden Inflammin, CHP, LASP-1, GNAT, Mutarotase, CPS 1 sowie den Peptid-Prohormonen proANP, proBNP, proADM und dem C-reaktiven Protein (CRP).

10. Verfahren nach Anspruch 8 oder 9, **dadurch gekennzeichnet, dass** die Multiparameter-Bestimmung als Simultanbestimmung mittels einer Chiptechnologie-Messvorrichtung oder einer immunchromatographischen Messvorrichtung erfolgt.

11. Verfahren nach Anspruch 10, **dadurch gekennzeichnet, dass** die Auswertung des mit der Messvorrichtung gewonnenen komplexen Messergebnisses mit Hilfe eines Computerprogramms erfolgt.

12. Verfahren nach einem der Ansprüche 1 bis 5, **dadurch gekennzeichnet, dass** es als immunchromatographisches Point-of-Care Verfahren (Schnelltest) durchgeführt wird.
